# EUROPEAN PATENT SPECIFICATION

(11) **EP 1 317 489 B1**
(45) Date of publication and mention of the grant of the patent: **28.12.2005**
(21) Application number: 01964775.9
(22) Date of filing: 11.09.2001
(51) Int. Cl.: C08B 37/00, C12P 19/00, A61K 7/48, A61K 31/715

(54) **A MIXTURE OF NON-SULFATED FUCOSE-BASED OLIGOSACCHARIDES, A COSMETIC OR PHARMACEUTICAL COMPOSITION COMPRISING SAID MIXTURE AND ITS USE IN COSMETICS OR PHARMACY**
EINE MISCHUNG VON NICHT-SULFATIERTEN OLIGOSACCHARIDEN AUF DER BASIS VON FUKOSE, EINE DIESE MISCHUNG ENTHALTENDE, KOSMETISCHE ODER PHARMAZEUTISCHE ZUSAMMENSETZUNG UND DEREN VERWENDUNGEN IN DER KOSMETIK UND PHARMAZIE
MELANGE D'OLIGOSACCHARIDES A BASE DE FUCOSE NON-SULFATE, COMPOSITION COSMETIQUE OU PHARMACEUTIQUE COMPORTANT LEDIT MELANGE ET SON UTILISATION DANS L'INDUSTRIE COSMETIQUE OU PHARMACEUTIQUE

(30) Priority: 11.09.2000 FR 0011545; 13.03.2001 BR 0100955
(43) Date of publication of application: 11.06.2003
(73) Proprietor: Industria E Comércio de Cosméticos Natura Ltda., CEP-0007750-000 Cajamar, SP (BR)
(72) Inventor: Robert, Ladislas, 94440 Santeny (FR); Robert, Alexandre Michel, 94440 Santeny (FR); Robert, Catherine Sylvie, 94449 Santeny (FR); Gesztesi, Jean-Luc, Sao Paulo, SP (BR)
(74) Representative: Brady, Paul Andrew
(86) International application number: PCT/BR2001/000114
(87) International publication number: WO 2002/020622

(56) References cited:
- EP-A- 0 818 201
- WO-A-96/23057

## Description

The present invention relates to a new mixture of non-sulfated fucose-based oligosaccharides and to its use mainly in products of topical application, for which an activity on the epithelial or conjunctive tissue is sought, in particular in products against aging, such as pharmaceutical and veterinary products and, more particularly, in cosmetic products.

While aging the skin becomes thin by approximately 6% on an average every ten years *(Skin thickness changes in normal aging skin,* Branchet et al, Gerontology, 1990, 36: 28-35). The underlying mechanisms of aging are intrinsic and extrinsic. The intrinsic mechanisms comprise a decrease in cellular proliferation and an important loss of the cutaneous extramolecular matrix (CEM). This loss is mostly the result of the drop in the synthesis of the CEM and of the increasing synthesis of degradation enzymes that can attack the skin matrix, with the aging (D.L.ROBERT: aging, CNRS, Belin, 1994; Docteur L. ROBERT: aging, facts and theories, DOMINOS, Flammarion, 1995). The responsible proteases are essentially the matrix metalloproteases (MMP), several of which various (such as serines-proteases) may degrade most of the constituents of the skin matrix and, in particular, the elastic fibers.

In the last few years, numerous researches have been made with the objective of obtaining active compounds against certain effects of skin aging. One first objective is to render this process slower. Another objective is to obtain a result of thickening the skin, mainly the dermis.

The saccharides and polysaccharides are well-known substances in cosmetics, mainly for their hydrating properties. This is the case, for instance, of the monosaccharide fucose and of the polysaccharides that contain it.

Fucose is a deoxy-hexose close to galactose, of which it has the steric conformation. However, the structure of fucose essentially differs from that of galactose in that the C6 atom bears a methyl group (-CH₃) and not a primary alcohol group (-CH₂OH). Indeed, this methyl group imparts to the molecule of fucose an interesting partial hydrophobic nature, compensated for by the hydroxyl groups on which other atoms on the four carbon atoms present.

Fucose appears early during the phylogenesis, the polysaccharides of certain algae and of fungi that contain them in relatively important quantities, either alone or in combination with other chemical compounds. It may equally appear in sulfated form like the fucanes. On the other hand, fucose is widespread in the animal kingdom and certain bacteria also synthesize it.

However, in spite of the considerable number of papers on fucose and the polysaccharides that contain it, the results achieved so far have almost not been satisfactory for application on an industrial scale, mainly in a cosmetic product.

In particular, in addition to the poor cost/effectiveness relationship of the monosaccharide fucose, the known polysaccharides containing fucose do not present a significant activity, which enables one to fight against the skin aging, as set forth above.

Thus, for instance, fucanes are sulfated polymers with high molecular weight (>20kD). WO 99/32099 (IFREMER) describes new uses of fucanes within the scope of repair of injuries of the conjunctive tissue. However, fucanes, due to their size and load, cannot interact effectively with all the cellular layers of the skin. In addition, fucanes can, on the contrary, activate the MMP-2, a redhibitory property for the cosmetic treatment of the normal aging of the skin.

FR-2 750 863 (L'ORÉAL) describes the use of a polyholoside, mainly a polysaccharide comprising fucose to favor scaling, that is to say, to favor the elimination of the "fly" cells located on the surface of the corneous layer of the epidermis (page 3, lines 6 and 7) and/or to stimulate the renewal of the epidermis, that is, to bring about forced elimination of the corneous layer that accelerates the renewal (page 1, lines 13 - 23), these effects bringing an assimilated result according to FR-2 750 863 to a result against skin aging. However, the polysaccharide as described in FR-2 750 863, for its molecular mass and its structure, does not allow one to integrate in a satisfactory way with the different constituents of the skin.

Then, it has been found, in an entirely surprising and unexpected way, that a new mixture of oligosaccharides containing fucose, which can be obtained by a specific treatment of the selected microorganism, presents significant activities on different components of the skin, bringing a real anti-aging result, clearly visible, mainly by an excellent thickening of the skin.

Thus, the present invention has the objective of providing a mixture of non-sulfated fucose-based oligosaccharides, characterized in that it comprises oligosaccharides of less then 13 saccharide units, comprising at least one fucose unit in a non-reducing end position, and in that it can be obtained by means of a process comprising at least one step of degradation of a polysaccharide from a microorganism of the gender *Klebsiella pneumoniae subsp. pneumoniae.*

By "non-sulfated fucose-based oligosaccharide" according to the invention, one understands, in accordance with the general knowledge of a person skilled in the art, an oligosaccharide containing at least one fucose saccharide unit and not bearing a sulfate group -O(SO3)⁻. Fucanes are, in particular, excluded from this definition.

By "oligosaccharide comprising at least one fucose unit in a non-reducing end position", according to the invention, one understands, in accordance with the general knowledge of a person skilled in the art, an oligosaccharide containing at least one fucose saccharide unit in an end position of the oligosaccharide chain, this fucose unit being linked to the next saccharide unit of the rest of the oligosaccharide by an acetal-type linkage.

The numbers of saccharide units may be measured with the aid of techniques known to a person skilled in the art, in particular, by applying the chromatography HPLC technique as described in the following examples.

Preferably, the mixture of oligosaccharides of the invention comprises, with respect to the total weight of the mixture, at least 15% by weight and, more particularly and preferably, from 20 to 50% by weight of oligosaccharides of less than 13 saccharide units, comprising at least one fucose unit in a non-reducing end position.

More particularly, the mixture of oligosaccharides, according to the invention, is characterized in that it further comprises, with respect to the total weight of the mixture, from 25 to 45% by weight of oligosaccharides having from 13 to 24 saccharide units, comprising at least one fucose unit in a non-reducing end position.

Still more particularly, the mixture of oligosaccharides of the invention is characterized in that it further comprises, with respect to the total weight of the mixture, from 15 to 35% by weight of oligosaccharides of more than 54 saccharide units, comprising at least one fucose unit in a non-reducing end position.

The mixture of oligosaccharide is capable of being achieved by means of a process that comprises at least one step of degradation of a polysaccharide from a microorganism of the gender *Klebsiella pneumoniae subsp. pneumoniae,* the oligosaccharides preferably comprising, at least in part, the motif of fucose-galactose galacturonic acid.

In particular, the mixture of oligosaccharides, according to the invention, is capable of being achieved by the process the comprises the steps of:
a) causing the microorganism of the gender *Klebsiella pneumoniae subsp. pneumoniae* to grow in an aqueous nutritive medium by aerobic fermentation of an assimilable source of glucide;
b) recovering the polysaccharide formed from the fermentation must;
c) subjecting the formed polysaccharide to a moderate hydrolysis;
d) subjecting the hydrolysis product of the step c) to an enzymatic hydrolysis; and
e) deactivating the enzyme after recovering the mixture of oligosaccharides thus formed.

More particularly, these steps a) - e) may be described in the following way:

### Step a)

One preferably uses the microorganism *Klebsiella pneumoniae subsp. pneumoniae,* which is the microorganism deposited in the Collection Nationale de Cultures de Microorganismes under the number I-1507, or a mutant thereof. On the other hand, this microorganism is described in detail in application WO 96/23057.

The aqueous nutritive medium may be any aqueous medium known to a person skilled in the art that contains sources of carbon, nitrogen and mineral salts, such as those described in application WO 96/23057.

The fermentation may be conducted at temperatures on the order of from 25 to 35°C, with a pH of about 6.0 to 7.5, under conditions of aeration and stirring, for periods of from 2 to 4 days.

The fermentation may be made in a classic fermenter, inoculating the previously sterilized nutritive medium, for example, by heating up to a temperature of about 120°C or by sterilizing filtration.

### Step b)

At the end of the period of fermentation, the fermentation must is recovered, and a fucose-rich polysaccharide is isolated from it in the following way:

The fermentation must is subjected to a heat treatment at a temperature specially ranging from about 100 to about 130°C, preferably from about 115 to about 125°C, for about 30 minutes to about 2 hours and, preferably, from about 40 minutes to about 1 hour and with a pH specially ranging from 2 to about 5.5 and, preferably, from about 3 to about 5.5.

The product of the heat treatment is filtered according to classic means such as a press filter with plates.

In this way, one obtains a limpid, viscous polysaccharide, free from any cell.

Then a precipitation is carried out in an alcohol solvent, preferably an alcohol solvent chosen from ethanol, isopropanol and mixtures thereof. In particular, one uses from about 1 to about 3.0 volumes of solvent to 1 volume of polysaccharide and, preferably, from about 1.3 to about 2.0 volumes of solvent to 1 volume of polysaccharide.

Then, one carries out the drying under vacuum at a temperature specially ranging from about 20 to about 60°C and, preferably, from about 30 to about 50°C, until a powder is obtained.

### Steps c) and d): hydrolysis of the polysaccharide

This is an essential combination of steps. Indeed, one has found, in a surprising and unexpected way, that the combination of a step of moderate hydrolysis, preferably by irradiation with gamma rays and/or by protolysis, with an enzymatic hydrolysis step, enables one to obtain advantageously a sufficient global output of hydrolysis, close to that of a classic hydrolysis, such as an acidic hydrolysis, but with the advantage of specific cuts of an enzymatic hydrolysis. In particular, an acidic classical hydrolysis does not enable one to obtain a mixture of specific oligosaccharides, according to the invention, as it leads to the obtention of statistic, redhibitory cuts, as to the random nature. In addition, the compounds resulting from a classic acidic hydrolysis prove to be biologically inactive.

### Step c): moderate hydrolysis of the polysaccharide

The moderate hydrolysis is carried out by a treatment with gamma rays, a protolysis treatment or by these two successive treatments. Preferably, one successively carries out a treatment with gamma rays and then a protolysis treatment.

The treatment with gamma rays proved to cause a sensible drop in viscosity by a limited degradation, attributable to the action of free radicals. It may be carried out with irradiation means known to those skilled in the art.

This treatment by gamma rays, which are very penetrating rays, presents, in addition, the advantage of sterilizing the polisaccharide, killing the germs present, which could induce inflammation or even cause granuloma. In this way, one prevents a bacterial attack, without having to add to the medium any antiseptic products that could interfere in an undesirable way with the biologic activities of the end product.

The polysaccharide powder obtained in step b), possibly irradiated with gamma rays, may therefore, equally, be subjected to a protolysis treatment. For this purpose, it is placed in an aqueous solution, specially at the proportion of from 1 to 20% by weight and, preferably, from 2 to 10% by weight, with respect to the total weight of the aqueous solution.

The aqueous solution is subjected to a heat treatment, that is to say, a heating up to a temperature specially ranging from about 75 to about 120°C and, preferably, from about 90 to about 100° C, for a period of time ranging from 1 to 6 hours, in the presence of a proton-generating resin, such as those commercialized and well known to a person skilled in the art, that is to say, a resin generating protons that bring about a cut of the glucosidic linkages with fixation of a water molecule.

### Step d): enzymatic hydrolysis

One introduces an acidic buffer such as a citric acidic buffer (4.15 g/kg)- disodium hydrogenophosphate (about 10.75 g/kg) in the hydrolysate obtained in step b). One regulates the temperature of the solution specially to a temperature ranging from about 25 to about 45° C and, preferably from about 30 to about 40°C.

One introduces an enzymatic preparation comprising at least one endofucosidase, preferably Fermizyme HCP such as commercialized by Gist Brocades, according to contents specially from about 2 to about 20% by weight and, preferably, from about 5 to about 15% by weight, with respect to the initial weight of polysaccharide powder utilized.

The thus obtained mixture is maintained under stirring for a period of time ranging from about 8 to about 24 hours and, preferably, from about 10 to about 20 hours, at a temperature specially ranging from about 25 to about 45°C and, preferably, from about 30 to about 40° C, the pH being regulated at 6 by the presence of the buffer mixture.

### Step e)

The hydrolysis product obtained after the step d) is filtered according to classical means such as a press filter with plates.

The collected solution is then heat-treated at a temperature specially ranging from about 75 to about 120°C and, preferably, from about 90 to about 105°C, for a period of time specially ranging from about 10 to about 45 minutes and, preferably, from about 20 to about 35 minutes, in order to deactivate the enzyme and, more particularly, the fucosidase activity of this specific enzyme.

One let it cool down to a temperature specially ranging from about 20 to about 40°C.

While it is cooling, preservatives may by added to the solution.

One then filters the whole under sterile conditions, and then the packaging is carried out.

The thus obtained mixture of oligosaccharides according to the invention may be characterized with the aid of techniques well known to those skilled in the art, specially HPLC, chromatography on the thin layer and other methods and chemical dosages.

In this way, one can find out that the oligosaccharides of the mixture, according to the invention, are such that fucose is mainly at the end of the chain in a non-reducing end position.

The mixture of oligosaccharides is particularly suitable as an active mixture in a cosmetic composition that is specially anti-aging or in a pharmaceutical composition that is specially dermatological (topical application). In particular, the biologic effects found in this mixture of oligosaccharides are very comparable and even superior to those of the monosaccharide fucose.

Thus, the present invention equally has the objective of providing a cosmetic or pharmaceutical composition characterized by comprising, as a cosmetically or pharmaceutically active agent, at least one mixture of oligosaccharide such as described above and at least one cosmetically or pharmaceutically acceptable excipient.

When the composition is a pharmaceutical one, it is preferably a dermatological composition, for topical application, thus having at least one pharmaceutical excipient suitable for this dermatological application.

The cosmetically or pharmaceutically acceptable excipient may be any one from those known to a person skilled in the art for the purpose of obtaining a composition according to the invention in the form of a cream, a lotion, a gel, a salve, etc., possibly in the form of an emulsion, having, in addition, other components known to a person skilled in the art, to improve, modify or stabilize the composition from a cosmetic or pharmaceutical point of view.

The expression "pharmaceutically acceptable excipient" embraces excipients adapted for a veterinary use of the composition, according to the invention.

The composition according to the invention may, in particular, contain other additives and, as an aid to the formulation, such as antioxidant agents for fighting free radicals. One can cite specially pure vitamin E or di-alpha-tocopherol and its derivatives, and 2,6-di-tert-butyl-p-cresol (BHT).

Advantageously, the composition according to the invention may further comprise, in particular, at least one additive chosen from the group consisting of the agents structuring the skin (such as squalane and sphingo-lipides), the moistening agents (such as glycerin and hydroxy prosilan C), the emollients (such as butylene glycol and cetyl lactate), the silicones (such as cyclomethicone), the sun protection agents (such as Parsol 1789 and Eusolex 6300), the emulsifiers (specially Carbopol 1342 associated to triethanolamine and soybean lecithin), the thickeners (notably xanthan gum), the scavengers (specially EDTA), the antioxidants (such as BHT described above), the fragrances, the preservatives, water and mixtures thereof.

Of course, the operational conditions for preparing the cosmetic or pharmaceutical composition according to the invention are part of the general knowledge of the art.

Preferably, the mixture of oligosaccharides is present according to a proportion ranging from about 0.001 to about 20% by weight and, more particularly and preferably, from about 0.1 to about 10% by weight with respect to the total weight of the cosmetic or pharmaceutical composition.

However, without wanting to be trapped by any theory, the advantageous activity of the mixture of oligosaccharides according to the invention, illustrated in the examples given hereinafter, would result, in particular, from its very great capacity of interacting with the cellular membranes via the hydrophobic functions of the highly available methyl groups of the fucose, and from its interaction with the mannose-fucose receptor of the skin cells, macrophages or Langerhans cells (Condaminet et al: *Human epidermal Langerhans cells express the mannose-fucose binding receptor,* Eur. J. Immuno. 1998, 28: 3541-3551). The set of these effects may be characterized as an effect stimulating cellular communication.

Thus, the present invention has also the objective of using the mixture of oligosaccharides, as described above, for preparing a compos i-tion intended to stimulate cellular communication between the cells of the skin.

On the other hand, the present invention has also the objective of using the mixture of oligosaccharides as described above, for preparing a composition intended to stimulate the cellular proliferation of the keratinocytes of the skin.

In addition, the present invention has the objective of using the mixture of oligosaccharides, as described above, for preparing a compos i-tion intended to stimulate the cellular proliferation of the fibroblasts of the skin.

Finally, the present invention has the objective of using the mixture of oligosaccharides as described above, for preparing a composition intended to inhibit the synthesis of elastase-type proteases by the fibroblasts of the skin.

The fibroblasts of the human skin synthesize various proteases, some of which have an elastase-type activity. This is particularly true for MMP-2 and MMP-9 and for membrane MMP.

Hyaluronane added to the fibroblasts in low concentrations (1 - 2 mg/ml) increases the elastase-type activity significantly. Surprisingly and unexpectedly, we can see that the mixture of oligosaccharides according to the invention inhibits this stimulus. Since hyaluronane is constantly present in the pericellular environment, this inhibiting activity may be considered to enabling one to reduce the degradation of the skin matrix with the aging.

Thus, the present invention has equally the objective of using the mixture of oligosaccharides, as described above, for preparing a composition intended to inhibit the superexpression of the proteases MMP-2 and MMP-9, induced by hyaluronane.

More particularly, the present invention has also the objective of using the mixture of oligosaccharides, as described above, for preparing a composition intended to inhibit the synthesis of the proteases MMP-2 and MMP-9 by the fibroblasts of the skin.

On the other hand, it has been shown recently that the activity of the MMP is critical in the case of sensitizing by contact (M.C. Lebre et al, Arcg. Dermatol. Res., 1999, 291: 447-452). In the presence of MMP inhibitors, the Langerhans cells of the epidermis cannot migrate through the dermis, as is the case at the time of sensitizing by contact. In this way, inhibition of the activity of the MMP by the mixture of oligosaccharides, according to the invention, offers a real effect of decreasing the sensitivity of the skin to irritation.

Therefore, it is also an objective of the present invention to use the mixture of oligosaccharides, as described above, for preparing a composition intended to decrease the sensitivity of the skin to irritation.

The mixture of oligosaccharides according to the invention enables one to achieve effects of increasing the thickness of the epidermis and of the dermis. Statistically, these two effects are highly significant. When the thickness of the skin decreases with the aging, this trophic effect of the mixture of oligosacchariges according to the invention is extremely important in preventing skin aging.

More particularly, one can observe a reinforcement of the bundles of collagen of the dermis of a skin treated with the mixture of oligosaccharides according to the invention. Considering the importance of the role of these bundles of collagen in the steadiness of the skin, this is a greater anti-aging result.

Consequently, the present invention has also the objective of using the mixture of oligosaccharides, as described above, for preparing a composition intended to stimulate the deposit of collagen fibers on the dermis.

The mixture of oligosaccharides is used as described above, preferably according to a proportion ranging from about 0.001 to about 20% by weight and, more particularly and preferably, from about 0.1 to about 10% by weight, based on the total weight of the composition.

The thus prepared composition comprises, in addition, a cosmetically or pharmaceutically (specially dermatologically) acceptable excipient, such as those known to a person skilled in the art.

Finally, the present invention further has the objective of providing a method of cosmetic treatment of the skin, characterized by applying onto the skin a cosmetic composition comprising at least one mixture of oligosaccharides as described above. The cosmetic composition comprises, in addition, a cosmetically acceptable excipient, such as those known to a person skilled in the art.

The following examples are intended to illustrate the present invention and should not at all be interpreted as limiting its scope.
Figure 1 is a histogram reporting the results presented in example 3.b.1, in terms of percentage of effectiveness for reducing the active form/inactive form relationship of the MMP-2 and MMP-9 secreted by explants of the skin.
Figure 2 is a histogram reporting the results presented in example 3.b.1, in terms of percentage of effectiveness for reducing the active form/inactive form relationship of the MMP2- and MMP-9 secreted by explants of skin stimulated by hyaluronane.
Figure 3 is a histogram reporting the results represented in example 3.b.2, in terms of percentages of effectiveness for reducing the expression of the MMP-2 of explants of skin.

### Example 1: preparation of a mixture of oligosaccharides according to the invention

### a) Fermentation

One uses the microorganism *Klebsiella pneumoniae subsp. pneumoniae,* which is the microorganism deposited in the Collection Nationale de Cultures de Microorganismes under number I-1507. The nutritive medium and other fermentation conditions are as follows:
Preparation of the *inoculums:*
   Culture Medium:
      Neosorb® 70-07 (sorbitol contents: 70% M.S.; sold by ROQUE-TTE FRERES, Lille/France): 17.90 g/l (namely 12.5 g/l of sorbitol)
      Peptona Biokar 104003 (protein hydrolysate, sold by SOLABIA-BIOKAR, Pantin, France): - 4.50 g/l
      Yeast extract: 0.05 g/l
      KH₂PO₄: 1.50 g/l
      K₂HPO₄: 4.50 g/l
      MgSO₄,7H₂O: 0.20 g/l
      Pluronic® PE 61000 (antifoaming agent), sold by BASF, D-6700 Ludwigshafen, Germany: 0.50 g/l
      Placing in solution into water
   Culture Condition:
      Sterilization at 121°C for 30 minutes
      Culture temperature: 30°C
      Inoculation rate : 5 - 10%
      Aeration: 1 WM
      Non-regulated pH (pH approximately 7.00)
      Culture Duration: 24 hours
   Production Medium:
      Culture Medium
      Neosorb ® 70-07: 54.00 g/l (namely 38 g/l of sorbitol)
      Peptona Biokar ®b 104003: 4.50 g/l
      Yeast extract: 0.05 g/l
      KH₂PO₄: 1.50 g/l
      MgSO₄, 7H₂O: 0.20 g/l
      Pluronic ® PE 61000: 0.50 g/l
      Placing in solution into water
   Culture conditions (Chemap fermenter having useful volume of 350 liters):
      Sterilization at 120°C for 45 minutes
      Culture temperature: 30°C
      Inoculation rate: about 5%
      Stirring: 300 rpm (Rushton-type stirrer)
      Aeration: 1 VVM
      pH regulated at 7.0 by NaOH 7N
      Pressure: 100 - 200 mbars
      Culture Duration: 60 - 65 hours
   Average values achieved in production:
      Viscosity at the end of cycle: 40000 MPa.s (viscosimeters: Brookfield DV-II+model LV, movable SP 31, chamber SC4-34/13R, 30°C)
   Concentration of the polysaccharide produced in the medium, calculated in L-fucose:
      2g/l (Dische and Shettles methods)
      Sorbitol consumed: >35 g/l (in sorbitol)
      NaOH at 20% by weight consumed: 15 liters /m³
      Start of regulation of the pH: 16 - 17 hours, after inoculation of the fermenter
      Final dry extract of the fermentation medium ~20 g/l

### b) Recovery of the formed polysaccharide

One subjects the fermentation must to a heat treatment at a temperature of 120°C for 45 minutes and with a pH of 5.5. The product of the heat treatment is filtered with the help of a press filter with Seitz-type plates. In this way, one obtains a limpid, viscous polysaccharide, free from any cell. Then one carries out a precipitation in 1.5 volume of ethanol to 1 volume of polysaccharide. Then a drying is carried out under vacuum at a temperature of 25°C until a powder is obtained. Considering the microorganism used, this polysaccharide is composed of repetitive units of trisaccharide fucose - galactose - galacturonic acid, and thus it presents the following structure:

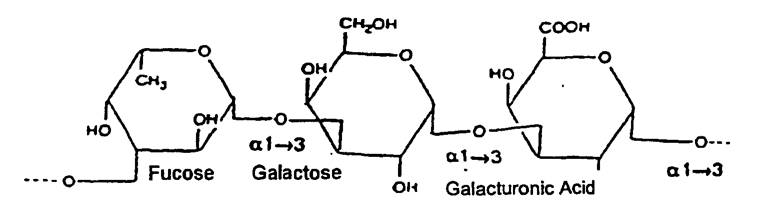

### c) Moderate hydrolysis of the polysaccharide

The polysaccharide powder is placed in aqueous solution at the proportion of 5% by weight, based on the total weight of the aqueous solution. The aqueous solution is subjected to a heat treatment, that is to say, heating up to 100° C, for 3 hours, in the presence of a proton-generating resin.

### d) Enzymatic hydrolysis

One introduces the buffer mixture citric acid (4.15 g/kg)-disodium hydrogenphosphate (about 10,75 g/kg) into the hydrolysate. The temperature of the solution is regulated at 37°C. One introduces the enzymatic preparation Fermizyme HCP, as commercialized by Gist Brocades, according to contents of 20% by weight, based on the initial weight of polysaccharide used, that is to say, 0,05% by weight with respect to the total mass of the aqueous solution after placing the powder into water again, as described above for the moderate hydrolysis by protolysis.

The thus obtained mixture is kept under stirring for 15 hours at a temperature of 37°C, the pH being regulated at 6 by the presence of the buffer mixture.

### e) Deactivation of the enzyme and recovery of the mixture of oligosaccharides

The product of hydrolysis is filtered with a press filter with Seitz-type plates. The solution collected is then heat-treated at 10° C, for 30 minutes, to deactivate the enzyme. One lets it cool at a temperature of 25° C. When it is cooling, the preservatives phenoxy ethanol (1% by weight) and phenonipe (0.3% by weight) are added to the solution. Then the whole is filtered in sterile conditions.

The thus obtained mixture of oligosaccharides is called "Mixture-1".

### EXAMPLE 2: characterization HPLC of the Mixture-1

The fractionation of the Mixture-1 obtained in example 1 was carried out for the purpose of determining the proportion of oligosaccharides and polysaccharides in its composition.

### a) Fractionation by Preparative Exclusion Chromatography on column "XK 50/60 Superdex 75 prepgrade"

One passes 50 ml of the Mixture-1 concentrated with 50 mg/ml, on a preparative column "XK 50/60 Superdex 75 prepgrade" (exclusion chromatography) and collects 95 fractions, then passed in HPLC.

**Table 1:**

| technical informations referring to the preparative column XK 50/60 Superdex 75 prepgrade | |
|---|---|
| Packing | Superdex 75 prepgrade (34 µm) |
| Column size | Height: 50 cm Diameter: 60 mm |
| Column type | XK 50/60 |
| Usable interval of fractionation | 5 x 10² Da-3 x 10⁴ Da |
| Injected sample | 50 ml of the mixture-1 at concentration of 50 mg/ml (total: 2.50 g) |
| Elution Speed | 1 ml/minute |
| Number of collected fractions | 95 fractions of 12.5 ml each |
| Movable phase | PBS |

After fractionation the Mixture-1 on the column "XK 50/60 Superdex 75 prepgrade", 95 fractions are collected, 45 of which contain osides.

### b) Characterization of the fractions obtained by HPLC (exclusion chromatography), ultrahydrogel 120 and ultrahydrogel 250 columns

The objective of this second part of the study was to pass all the 95 fractions of the mixture-1 on a HPLC exclusion column (Ultrahydrogel 120 and 250 columns), in order to analyze the molecular weights and the concentration of the components of these fractions.

For this study one has worked with a Waters HPLC system, the description of which follows.

**Table 2:**

| technical characteristics of the HPLC chromatography system used. | |
|---|---|
| Apparatus | HPLC Waters 600 |
| Columns | Ultrahydrogel 120 (pore size: 120 Å) and Ultrahydrogel 250 (pore size: 250 Å) from Waters. Size: 7.8 mm x 300 mm, containing the gel of hydroxylated polymethacrylate |
| Injected samples | 20µl per automatic injector |
| Elution Time of elution | 0.10 M NaNO₃ 50 minutes/samples |
| Elution speed | 0.5 ml/minute |
| Detection | By measuring the refraction index with a Waters 410 refractometer |

**Table 3:**

| standards of molecular weights of polyethylene glycol (Fluka) used for the HPLC exclusion chromatography | |
|---|---|
| Molecular weight | Time of elution (minutes) |
| 400 | 37.892 |
| 600 | 36.026 |
| 1000 | 33.940 |
| 2000 | 31.154 |
| 4000 | 28.896 |
| 6000 | 27.868 |
| 8000 | 26.946 |
| 12000 | 26.192 |
| 20000 | 25.308 |
| 35000 | 24.216 |

### c) Results

The molecular weights of the components of the studied fractions were calculated, by using the following equation, obtained with the standards of molecular weights of Fluka, described in Table 3:
Molecular weight of the components = 55290000 *10^(-0.13942* x)
R² = 0.982
X = time of elution (minutes)

The first fraction containing components of the Mixture-1 is fraction No. 44 and the last one is fraction No. 89, which means that the same component of the less elevated molecular weight is obtained after 89 fractions collected (after an elution of 89 x 12.5 ml = 1112.5 ml). The fractions collected contain mono-, oligo- and polysaccharides of 184Da (mixture of monosaccharides) up to about 21 kDa. Therefore, this fraction contains polysaccharides formed by an average of 117 monosaccharide units or of 39 trisaccharide units.

Most of the fractions, with the exception of fractions No. 77, 78, 79, 81, 82, 83, 84, 85, and 86, contain a single saccharide peak (separation limited by the sensitivity of the separation method applied).

The approximate concentration of the different fractions may be determined by using an appraisal range of fucose standard at growing concentrations. This kind of "mono-compositional" appraisal range could be used thanks to the detection system (measure of the refraction index with a refractometer). According to these results, a solution of 1 µg/ml of fucose gives, on an average, a surface peak of 29409 (arbitrary units of the system). Knowing the surfaces of the peaks analyzed, it was possible to calculate their apparent concentrations.

The results achieved show that the Mixture-1 contains approximately 26% of small osides (up to 2 kDa, about 4 trisaccharide units), about 36% of oligosaccharides (up to 4 kDa, 8 trisaccharide units) and about 23% of polysaccharides of molecular weight higher than 10 kDa (18 trisaccharide units).

Considering the microorganism and the specific enzyme (endofucosidase) used for preparing the Mixture-1, it seems that the oligosaccharides of the mixture comprise a fucose unit in non-reducing end position.

Example 3: activity of the fucose and of the Mixture-1 on the activity of the matrix metalloproteases of human skin

Here one proposes to study the effect of the monosaccharide fucose and of the mixture of oligosaccharide "Mixture-1", as prepared in Example 1 above, on the activity of the matrix metalloprotease (or MMP) of the fibroblasts in the quiescent state or stimulated by hyaluronane. Tests were carried out from cellular cultures of fibroblasts of human skin cultivated in monolayers, but also from culture of explants for studying the effect of the different treatments on a cellular environment closest to those of the skin. Indeed, the dermal fibroblasts are involved by an extracellular matrix abundant *in vivo,* a culture of explant is, therefore, a type of culture that reconstitutes the natural environment of the cells, thus enabling one to study the effect of the treatments on the activity of MMP of the fibroblasts in conditions close to the physiological conditions.

### a) Method

### a.1) Culture of skin explant

### a.1.1) Activity MMP of the non-stimulated skin explants

Skin explants from a skin of the belly of a 54 years old woman were cultivated in a DMEM medium without phenol red (GIBCO) under stirring at 37°C for 48 hours with different treatments. The samples were treated with a concentration of 10 µg/ml.

Recovery of the samples:
Extracellular medium - 1 ml of medium
Intracellular medium -explant ground with *ultraturax* in 1 ml of MMP buffer
MMP Buffer Composition:
   0.1 M Tris-HCl, pH 7.5, 0.1 M NaCl, 10 mM CaCl₂, 1 mM zinc acetate
   0.01 % Brij 35, 0.01 % NaN₃
   Zymography
   Gelatin, substrate of the MMP-s, was co-polymerized at final 1 mg/ml with a gel of polyacrylamide at 10%. The extracellular media from the cultures of skin explant were deposited on the top of the gel, the electrophoresis was carried out at 150 volts. The gels were then incubated in MMP buffer for 24 hours under stirring at 37°C. The gels were then colored with Co-omassie blue, then uncolored with distilled water. The lise ranges corresponding to an MMP activity, appeared as white, while the background of the gel was blue. In function of the distance of migration of proteins, one could determine the molecular weights of the enzymes having digested the gelatin. The MMP activity was quantified by morphometric analysis with the aid of a Visiolab computer.

### a .1.2) MMP activity of the skin explants stimulated with 12 mg/ml of hyaluronane

The protocol of the studies was the same as that used for the study of the MMP activity of the non-stimulated skin explants, but with hyaluronane with a concentration of mg/ml and the samples of oligosaccharides with a concentration of 10 µg/ml, added in the culture medium.

### a .2) Culture of skin fibroblasts

### a 2.1) MMP activity of the non-stimulated skin fibroblasts

Skin fibroblast from a skin of the belly of a 45 years old woman were cultivated until the 7^{th} cellular passage in a DMEM Glutamax (GIBCO) medium containing 1% of antibiotics and of fungicide and 10% of fetal calf serum and were used for the experiment. The fibroblasts were inseminated on 6-well plates at a density of 5.10⁴ cells per well. When at the confluence, the culture medium was replaced by a DMEM medium, without phenol red containing 0.1 % of BSA and containing the different substances to be treated at a concentration of 10 µg/ml. After 24 hours of culture at 37°C (5% (v/v) CO₂, 95% (v/v) air) the samples were recovered.
Extracellular medium - 1 ml of medium + 2 rinsings with 0.25 ml of PBS
Intracellular medium - cellular carpet sonicated with 1 ml of MMP buffer
MMP Buffer composition 0.1 M Tris-HCl, pH 7.5, 0.1 M NaCl, 10 mM CaCl₂, 1 mM zinc acetate
0.01% Brij 35, 0.01% NaN₃

As in the case of the cultures of skin explants, the culture media of the fibroblasts were studied with zymography.

### a. 2.2) MMP activity of the fibroblasts of skin stimulated with 1 mg/ml of hyaluronane

The protocol of the studies is the same as that used for the study of the MMP activity of the non-stimulated fibroblasts of skin, but with hyaluronane with a concentration of 1 mg/ml and the samples of oligosaccharides with a concentration of 10 µg/m), added in the culture medium.

### b) Results

### b.1) MMP activity of the skin explants

The MMP-s are enzymes that are secreted in the form of zymogens, inactive. Their activation is effected by proteolytic cleavage, the active form of the enzyme may then degrade the compounds of the extracellular matrix. Therefore, it is important to know not only the level of expression of the MMP-s, but also the proportion of MMP present in active form, only capable *in vivo* of degrading the extracellular matrix, just as the enzymes capable of activate the latent forms. The results were therefore presented in the form of active form/inactive form relationship of the enzyme and the percentages in relation to the proof were calculated to show the effectiveness of the fucose and of the Mixture-1 on its capacity of decreasing the active proportion of the MMP and possibly to inhibit the activation of the inactive form.

**Table 4:**

| MMP activity of non-stimulated skin explants: Active form/inactive form relationship of the enzyme % of effectiveness of the treatments: reduction of this relationship | | | | |
|---|---|---|---|---|
| | Active/Inactive MMP-9 | % effectiveness | Active/Inactive MMP-2 | % of effectiveness |
| Control | 5.48 | - | 0.85 | - |
| Fucose 10µg/ml | 2.38 | 57% | 0.80 | 6% |
| Mixture-1 10µg/ml | 3.09 | 46% | 0.67 | 21% |

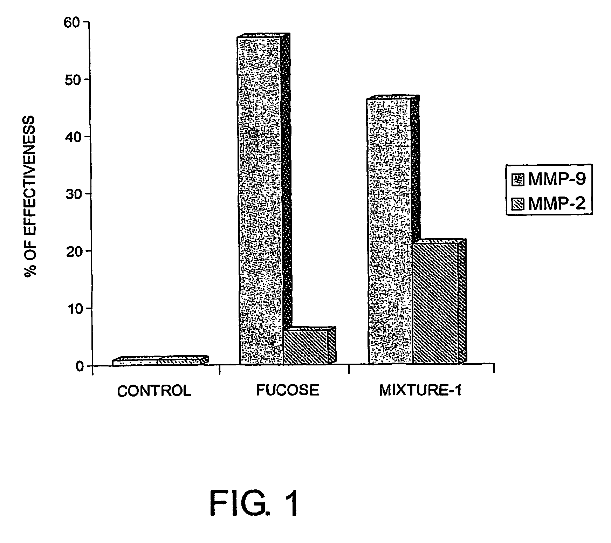

These results were equally presented in the form of histogram in figure 1.

Fucose and the Mixture-1 are capable of decreasing the active form/inactive form relationship of the enzyme. This phenomenon is more important for the MMP-9 than for the MMP-2. The decrease of this relationship implies that the amount of active enzyme, therefore capable of degrading in *vivo* the extracellular matrix, is less important when the skin explants are cultivated in the presence of fucose or the Mixture-1.

**Table 5:**

| MMP activity of skin explants stimulated with 1 mg/ml of hyaluronane | | | | |
|---|---|---|---|---|
| Active form/inactive form relationship of the enzyme % of effectiveness of the treatments: reduction of this relationship | | | | |
| | | | | |

| | Active/inactive MMP-9 | % effectiveness | Active/inactive MMP-2 | % effectiveness |
|---|---|---|---|---|
| Control | 7.08 | - | 1.58 | - |
| Fucose 10µg/ml | 3.54 | 50% | 1.65 | Ns |
| Mixture-1 10µg/ml | 1.98 | 72% | 0.98 | 38% |

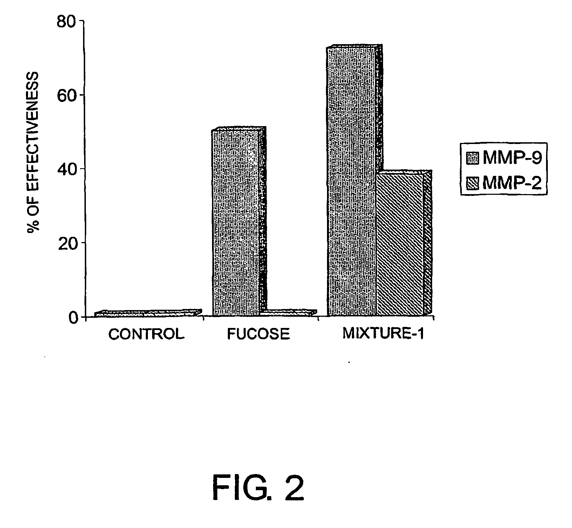

These results were equally presented in the form of histogram in figure 2.

The presence of hyaluronane in the culture medium of skin explants causes an increase in the active form of the MMP-s. Fucose and the Mixture-1 reduce this increase and are, therefore, capable of inhibiting this stimulation of release of active enzymes.

### b.2) MMP activity of the fibroblasts

When the fibroblasts are cultivated in monolayer, only the widely secreted MMP-2 is visible with a very low quantity of MMP-9, however, present. Only the inactive form of the MMP-2 enzyme is released.

**Table 6:**

| MMP activity of non-stimulated skin explants % of effectiveness of the treatments: reduction of the expression of MMP-2 | |
|---|---|
| | % of effectiveness |
| Control | - |
| Fucose 10µg/ml | 6% |
| Mixture-1 10µg/ml | 22% |

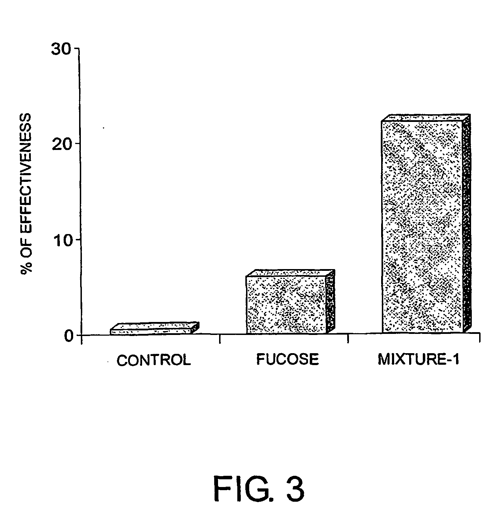

These results were equally presented in the form of the histogram of figure 3.

One can observe that fucose and the Mixture-1 decrease the expression of MMP-2. The Mixture-1 seems to be more effective than fucose for decreasing this expression.

**Table 7:**

| MMP activity of skin explants not stimulated with 1 mg/ml of hyaluronane | |
|---|---|
| % of effectiveness of the treatments: reduction of the expression of MMP-2 with respect to the control | |

| | % effectiveness |
|---|---|
| Control | - |
| Fucose 10 µg/ml | 11% |
| Mixture-1 10µg/ml | 30% |

### c) Conclusion

It follows from these experiments that fucose and the Mixture-1 are capable of decreasing the synthesis, as well as the activation of the MMP-2 and MMP-9 by the fibroblasts of human skin. In the same way, fucose and the Mixture-1 are capable of braking, in a more effective way, the superexpression of the metalloproteases in the presence of hyaluronane. Considering the important role of these metallo-endopeptidases in the degradation of the cutaneous extracellular matrix during the aging, this is a greater anti-aging effect.

### Example 4: activity of fucose and of the Mixture-1 on the fibroblasts of human skin

The objective of this study was to analyze the action of the Mixture-1 of example 1 on one of the characteristic parameters of the cellular communication and of the action of preventing aging of the fibroblasts of human skin, namely: stimulus of the cellular proliferation of the fibroblasts of human skin.

### a) Methodology: study of the cellular proliferation

The fibroblasts of human skin used in this study come from the removal of skin from a woman of 20 years old (26^{th} passing). The cells were cultivated on 12-well plates, in a DMEM culture medium with 10% of fetal calf serum (SVF), 1% of antibiotics and of antifungus (PSF), and 1 µCi/ml of [³H]-timidine (ICN) for 72 hours in the presence of the products to be tested with 2 final concentrations for each sample: 1 µg/ml and 10 µg/mL.

After 72 hours of culture in stove (5% (v/v) CO₂, 95% (v/v) air) at 37° C in the presence of samples, the cells were washed four times with PBS, then the cellular carpet was detached for 0.05% of tripsin. Three ml of scintillation liquid were then added per sample, then the radioactivity incorporated in the cells is read in a computer with scintillation.

### b) Results: action on the cellular proliferation

72-hour incubation of the fibroblasts with the two samples tested (concentrations of 1 µg/ml and 10 µg/ml) significantly stimulates the cellular proliferation in comparison with non-treated cells (see Table 8 below).

**Table 8:**

| effects of different concentrations of the Mixture-1 on the cellular proliferation of the fibroblast of human skin (proliferation with respect to the control) | | | |
|---|---|---|---|
| Product | Concentration | Effectiveness with respect to the control | P with respect to the control |
| Control | | | |
| Mixture-1 | 1 µg/ml | +48,2% | *** 0.000 |
| Mixture-1 | 10µg/ml | +30,8% | *** 0.002 |

### Example 5: cream against aging

| Component | % by weight |
|---|---|
| water | q.s.p. 100% |
| Sodium benzoate | 0.2 |
| Di-sodium EDTA | 0.08 |
| Glycerin | 2.00 |
| Butylene glycol | 4.00 |
| Carbomer ETD 2020 | 0.20 |
| Ceteareth-20 | 1.00 |
| Mineral oil | 3.00 |
| Squalane | 2.00 |
| Octyl palmitate | 6.00 |
| Karité butter ("Shea Butter") | 2.50 |
| Cetearyl alcohol | 1.00 |
| Rosa AFF Rubiginosa seed oil ("Seed Oil") | 0.20 |
| Decyl oleate | 0.50 |
| Octyl methoxycinamate | 5.00 |
| Butyl methoxydibenzoylmethane | 0.50 |
| BHA | 0.01 |
| Cyclomethicone | 5.00 |
| Cyclomethicone & Dimethiconol | 2.00 |
| Dimethicone | 2.00 |
| Fragrance (Crematest Feno) | 0.09 |
| Fragrance (Chemoderm) | 0.09 |
| Triethanolamine | 0.30 |
| 2-bromo-2-nitropropane-1,3-diol | 0.02 |
| | |
| Mixture-1 | 0.50 |

### Example 6: cream against aging

| Component | % by weight |
|---|---|
| Water | q.s.p. 100% |
| Sodium benzoate | 0.2 |
| Di-sodium EDTA | 0.08 |
| Glycerin | 2.00 |
| Butylene glycol | 4.00 |
| Carbomer ETD 2020 | 0,20 |
| Ceteareth-20 | 1.00 |
| Mineral oil | 3.00 |
| Squalane | 2.00 |
| Octyl palmitate | 6.00 |
| Karité butter ("Shea butter") | 2.50 |
| Cetearyl alcohol | 1.00 |
| Rosa AFF Rubiginosa seed oil | 0.20 |
| Ethearyl oleate | 0.50 |
| BHA | 0.01 |
| Cyclomethicone | 5.00 |
| Cyclomethicone & Dimethiconol | 2.00 |
| Dimethicone | 2.00 |
| Fragrance (Crematest Feno) | 0.09 |
| Fragrance (Chemoderm) | 0.09 |
| Triethanolamine | 0.30 |
| 2-bromo-2-nitropropane-1,3-diol | 0.02 |
| Mixture-1 | 0.50 |

## Claims

1. A mixture of non-sulfated fucose-based oligosaccharides, **characterized in that** it comprises oligosaccharides of less than 13 saccharide units, comprising at least one fucose unit in a non-reducing end position, and that it is capable of being obtained by means of a process that comprises at least one step of degradation of a polysaccharide from a microorganism of the gender *Klebsiella pneumoniae subsp. pneumoniae.*

2. A mixture of oligosaccharides according to claim 1, **characterized by** comprising, with respect to the total weight of the mixture, at least 15% by weight of oligosaccharides of less than 13 saccharide units, comprising at least one fucose unit in a non-reducing end position.

3. A mixture of oligosaccharides according to claim 1 or 2, **characterized by** comprising, with respect to the total weight of the mixture, from 20 to 50% by weight of oligosaccharides of less than 13 saccharide units, comprising at least one fucose unit in a non-reducing end position.

4. A mixture of oligosaccharides according to any one of the preceding claims, **characterized by** further comprising, with respect to the total weight of the mixture, from 25 to 45% by weight of oligosaccharides having from 13 to 24 saccharide units, comprising at least one fucose unit in a non-reducing end position.

5. A mixture of oligosaccharides according to any one of the preceding claims, **characterized by** further comprising, with respect to the total weight of the mixture, from 15 to 35% by weight of oligosaccharides of more than 54 saccharide units, comprising at least one fucose unit in a non-reducing end position.

6. A mixture of oligosaccharides according to any one of the preceding claims, **characterized in that** the oligosaccharides comprise, at least in part, the motif of recovery fucose-galactose-galacturonic acid.

7. A mixture of oligosaccharides according to any one of the preceding claims, **characterized in that** it can be obtained by the process that comprises the steps of:
a) causing the microorganism of the gender *Klebsiella* *pneumoniae subsp. pneumoniae* to grown in an aqueous nutritive medium by aerobic fermentation of an assimilable source of glucide;
b) recovering the polysaccharide formed from the fermentation must;
c) subjecting the formed polysaccharide to a moderate hydrolysis;
d) subjecting the hydrolysis product of the step c) to an enzymatic hydrolysis; and
e) deactivating the enzyme after recovering the mixture of oligosaccharides thus formed.

8. A mixture of oligosaccharides according to any one of the preceding claims, **characterized in that** the microorganism *Klebsiella pneumoniae subsp.pneumoniae* is the microorganism deposited in the Collection Nationale de Cultures de Microorganismes under number I-1507 or a mutant thereof.

9. A mixture of oligosaccharides according to claim 7 or 8, **characterized in that** the moderate hydrolysis is carried out by a treatment chosen from the group constituted by the treatments with gamma rays, by the protolysis treatments and by the combination of these treatments.

10. A mixture of oligosaccharides according to any one of claims 7 to 9, **characterized in that** the enzymatic hydrolysis is carried out with at least one endofucosidase.

11. A mixture according to claim 10, **characterized in that** the endofucosidase is Fermizyme HCP.

12. A cosmetic or pharmaceutical composition **characterized by** comprising, as a cosmetically or pharmaceutically active agent, at least one mixture of oligosaccharides according to any one of the preceding claims, and at least one cosmetically or pharmaceutically acceptable excipient.

13. A composition according to claim 12, **characterized in that** it is a dermatological composition.

14. A composition according to claim 12 or 13, **characterized in that** the mixture of oligosaccharides is present according to a proportion ranging from about 0.001 to about 20% by weight, based on the total weight of the composition.

15. Use of the mixture of oligosaccharides according to any one of claims 1 - 11, for preparing a composition intended to stimulate cellular communication between the cells of the skin.

16. Use of the mixture of oligosaccharides according to any one of claims 1 - 11, for preparing a composition intended to stimulate cellular proliferation of the keratinocytes of the skin.

17. Use of the mixture of oligosaccharides according to any one of claims 1 - 11, for preparing a composition intended to stimulate cellular proliferation of the fibroblasts of the skin.

18. Use of the mixture of oligosaccharides according to any one of claims 1 - 11, for preparing a composition intended to inhibit the synthesis of the elastase type proteases by the fibroblasts of the skin.

19. Use of the mixture of oligosaccharides according to any one of claims 1 - 11, for preparing a composition intended to inhibit the superexpression of the proteases MMP-2 and MMP-9 induced by hyaluronane.

20. Use according to claim 18 or 19, **characterized in that** the composition is intended to inhibit the synthesis of the MMP-2 and MMP-9 proteases by the fibroblasts of the skin.

21. Use according to claim 19 or 20, **characterized in that** the composition is intended to decrease the sensitivity of the skin to irritation.

22. Use of the mixture of oligosaccharides according to any one of claims 1 - 11 for preparing a composition intended to stimulate the deposit of collagen fibers on the dermis.

23. Use according to any one of claims 15 - 22, **characterized in that** the mixture of oligosaccharides is used according to a proportion ranging from about 0.001 to about 20% by weight, based on the total weight of the composition.

24. Use according to any one of claims 15 - 23, **characterized in that** the prepared composition further comprises a cosmetically or pharmaceutically acceptable excipient.

25. A method of cosmetic treatment of the skin, **characterized in that** one applies to the skin a cosmetic composition comprising at least one mixture of oligosccharides as defined in any one of claims 1 - 11.

26. A method of cosmetic treatment according to claim 25, **characterized in that** the cosmetic composition further comprises a cosmetically acceptable excipient.

## Patentansprüche

1. Mischung von unsulfatierten, auf Fucose basierenden Oligosacchariden, **dadurch gekennzeichnet, dass** sie Oligosaccharide von weniger als 13 Saccharid-Einheiten und mindestens eine Fucose-Einheit in einer nicht-reduzierenden Endposition enthält und dass sie durch ein Verfahren herstellbar ist, das mindestens einen Schritt des Abbaus eines Polysaccharid aus einem Mikroorganismus des Geschlechts Klebsiella pneumoniae subsp. pneumoniae umfasst.

2. Mischung von Oligosacchariden nach Anspruch 1, **dadurch gekennzeichnet, dass** mindestens 15 Gew.-%, bezogen auf das Gesamtgewicht der Mischung, Oligosaccharide mit weniger als 13 Saccharid-Einheiten und mindestens eine Fucose-Einheit in einer nicht-reduzierenden Endposition enthalten sind.

3. Mischung von Oligosacchariden nach Anspruch 1 oder 2, **dadurch gekennzeichnet, dass** von 20 bis 50 Gew,-%, bezogen auf das Gesamtgewicht der Mischung, Oligosaccharide mit weniger als 13 Saccharid-Einheiten und mindestens eine Fucose-Einheit in einer nicht-reduzierenden Endposition enthalten sind.

4. Mischung von Oligosacchariden nach einem der vorhergehenden Ansprüche, **dadurch gekennzeichnet, dass** zusätzlich von 25 bis 45 Gew.-%, bezogen auf das Gesamtgewicht der Mischung, Oligosaccharide mit 13 bis 24 Saccharid-Einheiten und mindestens eine Fucose-Einheit in einer nicht-reduzierenden Endposition enthalten sind.

5. Mischung von Oligosacchariden nach einem der vorhergehenden Ansprüche, **dadurch gekennzeichnet, dass** zusätzlich 15 bis 35 Gew.-%, bezogen auf das Gesamtgewicht der Mischung, Oligosaccharide mit mehr als 54 Saccharid-Einheiten und mindestens eine Fucose-Einheit in einer nicht-reduzierenden Endposition enthalten sind.

6. Mischung von Oligosacchariden nach einem der vorhergehenden Ansprüche, **dadurch gekennzeichnet, dass** die Oligosaccharide zumindest teilweise das Motiv der Wiederholung von Fucose-Galactose-Glacturonsäure enthält.

7. Mischung von Oligosacchariden nach einem der vorhergehenden Ansprüche, **dadurch gekennzeichnet, dass** sie durch das Verfahren mit den folgenden Schritten herstellbar ist:
a) der Mikroorganismus des Geschlechts Klebsiella pneumoniae subsp. pneumoniae wird in einem wässrigen Nährmedium durch aerobe Fermentation einer vergleichbaren Glucid-Quelle aufgezüchtet,
b) die aus dem Fermentations-Most gebildeten Polysaccharide werden wieder hergestellt,
c) die gebildeten Polysaccharide werden einer moderaten Hydrolyse unterworfen,
d) das Hydrolyseprodukt aus Schritt c) wird einer enzymatischen Hydrolyse unterworfen und
e) nach Wiederherstellung der Mischung der so gebildeten Oligosaccharide wird das Enzym deaktiviert.

8. Mischung von Oligosacchariden nach einem der vorhergehenden Ansprüche, **dadurch gekennzeichnet, dass** der Mikroorganismus Klebsiella pneumoniae subsp. pneumoniae der Mikroorganismus ist, der in der nationalen Sammlung der Mikroorganismuskulturen unter Nr. 1-1507 oder einer Mutation hiervon abgelegt ist.

9. Mischung von Oligosacchariden nach einem der Ansprüche 7 oder 8, **dadurch gekennzeichnet, dass** die moderate Hydrolyse durch eine Behandlung ausgewählt aus der Gruppe bestehend aus Behandlungen mit Gamma-Strahlen, Protolyse-Behandlungen und deren Kombinationen durchgeführt wird.

10. Mischung von Oligosacchariden nach einem der Ansprüche 7 bis 9, **dadurch gekennzeichnet, dass** die enzymatische Hydrolyse mit mindestens einer Endofucosidase durchgeführt wird.

11. Mischung nach Anspruch 10, **dadurch gekennzeichnet, dass** die Endofucosidase Fermizym HCP ist.

12. Kosmetische oder pharmazeutische Zusammensetzung, **dadurch gekennzeichnet, dass** sie ein kosmetisch der pharmazeutisch wirksames Mittel, mindestens eine Mischung von Oligosacchariden nach einem der vorhergehenden Ansprüche und mindestens einen kosmetisch oder pharmazeutisch verträglichen Hilfsstoff enthält.

13. Zusammensetzung nach Anspruch 12, **dadurch gekennzeichnet, dass** es eine dermatologische Zusammensetzung ist.

14. Zusammensetzung nach einem der Ansprüche 12 oder 13, **dadurch gekennzeichnet, dass** die Mischung der Oligosaccharide in einem Anteil im Bereich von etwa 0,001 bis etwa 20 Gew.-%, bezogen auf das Gesamtgewicht der Zusammensetzung, anwesend ist.

15. Verwendung der Mischung von Oligosacchariden nach einem der Ansprüche 1 bis 11 für die Herstellung einer Zusammensetzung, die die Stimulation der zellularen Kommunikation zwischen den Hautzellen beabsichtigt.

16. Verwendung der Mischung von Oligosacchariden nach einem der Ansprüche 1 bis 11 für die Herstellung einer Zusammensetzung, die für die Stimulation der zellularen Poliferation der Keratinozyten der Haut beabsichtigt ist.

17. Verwendung der Mischung von Oligosacchariden nach einem der Ansprüche 1 bis 11 für die Herstellung einer Zusammensetzung, die für die Stimulation der zellularen Proliferation der Fibroblasten der Haut beabsichtigt ist.

18. Verwendung der Mischung von Oligosacchariden nach einem der Ansprüche 1 bis 11 für die Herstellung einer Zusammensetzung, die für die Inhibierung der Synthese von Proteasen vom Elastase-Typ durch die Fibroblasten der Haut beabsichtigt ist.

19. Verwendung der Mischung von Oligosacchariden nach einem der Ansprüche 1 bis 11 für die Herstellung einer Zusammensetzung, die für die Inhibierung der Superexpression der Proteasen MMP-2 und MMP-9, ausgelöst durch Hyaluronan, beabsichtigt ist.

20. Verwendung nach einem der Ansprüche 18 oder 19, **dadurch gekennzeichnet, dass** die Zusammensetzung für die Inhibierung der Synthese der Proteasen MMP-2 und MMP-9 durch die Fibroblasten der Haut beabsichtigt ist.

21. Verwendung nach einem der Ansprüche 19 oder 20, **dadurch gekennzeichnet, dass** die Zusammensetzung für die Verringerung der Empfindlichkeit der Haut gegenüber Irritationen beabsichtigt ist.

22. Verwendung der Mischung von Oligosacchariden nach einem der Ansprüche 1 bis 11 für die Herstellung einer Zusammensetzung, die für die Stimulierung der Ablagerung von Collagen-Fasern auf der Dermis beabsichtigt ist.

23. Verwendung nach einem der Ansprüche 15 bis 22, **dadurch gekennzeichnet, dass** die Mischung von Oligosacchariden in einem Anteil im Bereich von etwa 0,001 bis etwa 20 Gew.-%, bezogen auf das Gesamtgewicht der Zusammensetzung, verwendet wird.

24. Verwendung nach einem der Ansprüche 15 bis 23, **dadurch gekennzeichnet, dass** die hergestellte Zusammensetzung zusätzlich einen kosmetisch oder pharmazeutisch verträglichen Hilfsstoff enthält.

25. Verfahren zur kosmetischen Behandlung der Haut, **dadurch gekennzeichnet, dass** man eine kosmetische Zusammensetzung enthaltend mindestens eine Mischung von Oligosacchariden nach einem der Ansprüche 1 bis 11 auf der Haut aufbringt.

26. Verfahren zur kosmetischen Behandlung nach Anspruch 25, **dadurch gekennzeichnet, dass** die kosmetische Zusammensetzung zusätzlich einen kosmetisch verträglichen Hilfsstoff enthält.

## Revendications

1. Mélange d'oligosaccharides à base de fucose non sulfaté, **caractérisé en ce qu'**il comprend des oligosaccharides à moins de 13 motifs saccharide, comprenant au moins un motif fucose dans une position terminale non réductrice, et **en ce qu'**il peut être obtenu au moyen d'un procédé qui comprend au moins une étape de dégradation d'un polysaccharide issu d'un microorganisme du genre *Klebsiella pneumoniae subsp. pneumoniae.*

2. Mélange d'oligosaccharides selon la revendication 1, **caractérisé en ce qu'**il comprend, par rapport au poids total du mélange, au moins 15 % en poids d'oligosaccharides à moins de 13 motifs saccharide, comprenant au moins un motif fucose dans une position terminale non réductrice.

3. Mélange d'oligosaccharides selon la revendication 1 ou 2, **caractérisé en ce qu'**il comprend, par rapport au poids total du mélange, de 20 à 50 % en poids d'oligosaccharides à moins de 13 motifs saccharide, comprenant au moins un motif fucose dans une position terminale non réductrice.

4. Mélange d'oligosaccharides selon l'une quelconque des revendications précédentes, **caractérisé en ce qu'**il comprend en outre, par rapport au poids total du mélange, de 25 à 45 % en poids d'oligosaccharides ayant de 13 à 24 motifs saccharide, comprenant au moins un motif fucose dans une position terminale non réductrice.

5. Mélange d'oligosaccharides selon l'une quelconque des revendications précédentes, **caractérisé en ce qu'**il comprend en outre, par rapport au poids total du mélange, de 15 à 35 % en poids d'oligosaccharides à plus de 54 motifs saccharide, comprenant au moins un motif fucose dans une position terminale non réductrice.

6. Mélange d'oligosaccharides selon l'une quelconque des revendications précédentes, **caractérisé en ce que** les oligosaccharides comprennent, au moins en partie, le motif de répétition du fucose-galactose-acide galacturonique.

7. Mélange d'oligosaccharides selon l'une quelconque des revendications précédentes, **caractérisé en ce qu'**il peut être obtenu par le procédé qui comprend les étapes consistant à :
a) faire croître le microorganisme du genre *Klebsiella pneumoniae subsp. pneumoniae* dans un milieu nutritif aqueux par fermentation aérobie d'une source assimilable de glucide ;
b) récupérer le polysaccharide formé dans le moût de fermentation ;
c) soumettre le polysaccharide formé à une hydrolyse modérée ;
d) soumettre le produit d'hydrolyse de l'étape c) à une hydrolyse enzymatique ; et
e) désactiver l'enzyme après la récupération du mélange d'oligosaccharides ainsi formé.

8. Mélange d'oligosaccharides selon l'une quelconque des revendications précédentes, **caractérisé en ce que** le microorganisme *Klebsiella pneumoniae subsp. pneumoniae* est le microorganisme déposé à la Collection Nationale de Cultures de Microorganismes sous le numéro I-1507 ou un mutant de celui-ci.

9. Mélange d'oligosaccharides selon la revendication 7 ou 8, **caractérisé en ce que** l'hydrolyse modérée est effectuée par un traitement choisi dans le groupe constitué par les traitements avec des rayons gamma, par les traitements par protolyse et par la combinaison de ces traitements.

10. Mélange d'oligosaccharides selon l'une quelconque des revendications 7 à 9, **caractérisé en ce que** l'hydrolyse enzymatique est effectuée avec au moins une endofucosidase.

11. Mélange selon la revendication 10, **caractérisé en ce que** l'endofucosidase est la Fermizyme HCP.

12. Composition cosmétique ou pharmaceutique **caractérisée en ce qu'**elle comprend, comme principe cosmétiquement ou pharmaceutiquement actif, au moins un mélange d'oligosaccharides selon l'une quelconque des revendications précédentes, et au moins un excipient cosmétiquement ou pharmaceutiquement acceptable.

13. Composition selon la revendication 12, **caractérisée en ce qu'**il s'agit d'une composition dermatologique.

14. Composition selon la revendication 12 ou 13, **caractérisée en ce que** le mélange d'oligosaccharides est présent dans des proportions allant d'environ 0,001 à environ 20 % en poids, sur la base du poids total de la composition.

15. Utilisation du mélange d'oligosaccharides selon l'une quelconque des revendications 1 à 11 pour préparer une composition destinée à stimuler la communication cellulaire entre les cellules de la peau.

16. Utilisation du mélange d'oligosaccharides selon l'une quelconque des revendications 1 à 11 pour préparer une composition destinée à stimuler la prolifération cellulaire des kératinocytes de la peau.

17. Utilisation du mélange d'oligosaccharides selon l'une quelconque des revendications 1 à 11 pour préparer une composition destinée à stimuler la prolifération cellulaire des fibroblastes la peau.

18. Utilisation du mélange d'oligosaccharides selon l'une quelconque des revendications 1 à 11 pour préparer une composition destinée à inhiber la synthèse des protéases de type élastase par les fibroblastes de la peau.

19. Utilisation du mélange d'oligosaccharides selon l'une quelconque des revendications 1 à 11 pour préparer une composition destinée à inhiber la surexpression des protéases MMP-2 et MMP-9 induite par l'hyaluronane.

20. Utilisation selon la revendication 18 ou 19, **caractérisée en ce que** la composition est destinée à inhiber la synthèse des protéases MMP-2 et MMP-9 par les fibroblastes de la peau.

21. Utilisation selon la revendication 19 ou 20, **caractérisée en ce que** la composition est destinée à diminuer la sensibilité de la peau à l'irritation.

22. Utilisation du mélange d'oligosaccharides selon l'une quelconque des revendications 1 à 11 pour préparer une composition destinée à stimuler le dépôt de fibres de collagène sur le derme.

23. Utilisation selon l'une quelconque des revendications 15 à 22, **caractérisée en ce que** le mélange d'oligosaccharides est utilisé dans des proportions allant d'environ 0,001 à environ 20 % en poids, sur la base du poids total de la composition.

24. Utilisation selon l'une quelconque des revendications 15 à 23, **caractérisée en ce que** la composition préparée comprend en outre un excipient cosmétiquement ou pharmaceutiquement acceptable.

25. Procédé de traitement cosmétique de la peau, **caractérisé par** l'application sur la peau d'une composition cosmétique comprenant au moins un mélange d'oligosaccharides tel que défini dans l'une quelconque des revendications 1 à 11.

26. Procédé de traitement cosmétique selon la revendication 25, **caractérisé en ce que** la composition cosmétique comprend en outre un excipient cosmétiquement acceptable.
